# EUROPEAN PATENT SPECIFICATION

(11) **EP 1 094 807 B1**
(45) Date of publication and mention of the grant of the patent: **23.04.2003**
(21) Application number: 99926687.7
(22) Date of filing: 05.07.1999
(51) Int. Cl.: A61K 31/355, A61P 17/02

(54) **VITAMIN E AND ESTERS THEREOF FOR USE IN THE TOPICAL TREATMENT OF MUCOSAL PATHOLOGIES**
VITAMIN E UND SEINE ESTER ZUR VERWENDUNG IN DER LOKALE BEHANDLUNG VON SCHLEIMHAUTERKRANKUNGEN
VITAMINE E ET SES ESTERS DESTINES A L'UTILISATION DANS UN TRAITEMENT TOPIQUE DE PATHOLOGIES DES MUQUEUSES

(30) Priority: 10.07.1998 IT MI981586
(43) Date of publication of application: 02.05.2001
(73) Proprietor: BIO.LO.GA. S.r.l., 31015 Conegliano (TV) (IT)
(72) Inventor: PANIN, Giorgio, I-45100 Rovigo (IT); ANNUNZIATA, Eleonora, I-35128 Padova (IT)
(74) Representative: Ferreccio, Rinaldo
(86) International application number: IB9901238
(87) International publication number: WO00002554

(56) References cited:
- EP-A- 0 380 367
- EP-A- 0 535 446
- WO-A-97/45098
- PATENT ABSTRACTS OF JAPAN vol. 098, no. 014 & JP 10 231243 A (SEKISUI CHEM CO LTD), 2 September 1998 (1998-09-02)
- PATENT ABSTRACTS OF JAPAN vol. 014, no. 211 & JP 02 048525 A (LEDERLE JAPAN LTD), 19 February 1990 (1990-02-19)
- PATENT ABSTRACTS OF JAPAN vol. 009, no. 082 & JP 59 216812 A (DOUWA YAKUSHIYOU), 6 December 1984 (1984-12-06)
- DATABASE WPI Week 9729 Derwent Publications Ltd., London, GB; AN 97-311148 XP002096860 & CN 1 104 502 A (HONGDA IND. GEN. CO.), 5 July 1995 (1995-07-05)

## Description

### Technical Field

In general, the present invention refers to the use of α-tocopherol (in racemic form d,1 or of pure enantiomer d or 1), also known as Vitamin E, and of the esters thereof with carboxylic acids and, in particular, of Vitamin E acetate in the therapeutic treatment of mucosal pathology.

### Background Art

As was shown in the previous patent application by the same applicant WO-A-97/45098, Vitamin E and its derivatives are substances which, by virtue of their alleged anti-oxidant and free radical-scavenging properties, are widely used in the cosmetic industry in the preparation of formulations for treating or preventing skin disorders. For these reasons, Vitamin E and its derivatives are often present in cosmetic formulations as "active" components with an anti-oxidant and anti-ageing effect in concentrations varying from 0.5 to 10%. In reality, the clinical significance of the anti-oxidant and anti-ageing action of Vitamin E at the skin level has never been outlined by controlled *in vivo* studies.

Compositions for topical use containing up to 20% by weight (preferably up to 10%) of Vitamin E, in association with other active ingredients, emulsifiers and excipients, are reported in application EP-A-0 158 090. In that application, it is claimed that such compositions are effective in the treatment of eczema, skin inflammations and irritations, skin allergies, excessive skin pigmentation and the like, although experimental support is provided only for the application of pomades containing 8% of non-esterified Vitamin E in the prevention of erythema following exposure to sunlight or radiation.

In application WO97/45098 cited above, the use of esters of Vitamin E, and particularly of Vitamin E acetate as such, is reported as a cosmetic product for topical application and as a unique ingredient in the production of medicaments for topical treatment of pathological skin conditions.

Research on the possible therapeutic actions of tocopherol esters applied topically in undiluted form has been carried on, and significant results have been obtained in the treatment of mucosal diseases.

The following discussion shall particularly refer to tocopherol acetate, as a preferred example of a compound according to the present invention.

### Disclosure of Invention

According to the present invention, tocopherol acetate may be used "as is" as a medicament for topical mucosal use on various types of mucosal epithelium by virtue of its properties of hydrophobic ointment with protective capabilities, which are of assistance in the re-epithelization or normalization of the epithelium altered by various pathological conditions.

Its oily nature and its physiological affinity for biological membranes constitute the theoretical background for considering tocopherol as equivalent to a phospholipid. Its molecular structure, with one nonpolar and one polar component, is actually similar to the molecular structure of a phospholipid. Conjectures may therefore be raised with regard to its innovative use as a medicament on the mucosal level, in the restoration and re-integration of the lipidic-phospholipidic component of the mucosal secretions, in cases where, due to various pathological situations, a qualitative and quantitative deficit of the secretions themselves, with a severe compromise of the lipid component, arises in various mucosae (e.g.: oral, nasal, ocular, vaginal, rectal mucosa).

The polar lipids in the secretions are important in the formation of a superficial film which prevents evaporation of the aqueous component or replace the aqueous component when it, too, is in deficit.

The principal advantages of the innovative use of tocopherol as a "neolipid" at the level of the mucosa are represented by its absolute affinity for other biological molecules, it being itself a physiological molecule normally present in human biological and cellular membranes and not a extraneous molecule like other substances used to date(e.g.: mineral oils, polyethylene glycols, polyalcohols), and by its molecular structure of a "polar oil" very similar to phospholipids and with an affinity thereto, and can therefore be integrated with the aqueous phase of the mucosal secretions so as to reconstitute the lipidic component of the latter when it becomes compromised for any of a number of reasons.

The use of tocopherol on the mucosa, as a substitute for the lipidic component, both at the cellular level and at the level of secretions, represents therefore an innovation.

Another fundamental advantage is that of being a "membrane molecule" and exerting two important biological activities on biological membranes: a «membrane stabilizing action» and a «membrane modulating action». Vitamin E incorporates in the phospholipids of the biological membranes thanks to its structural affinity, thus increasing the stability of the latter versus different (antigenic, inflammatory, chemico-physical) stimuli. This incorporation by affinity occurs both with the cell membranes of the different kinds of cells at mucous and submucous level (e.g. cells of the mucous epithelium, mast cells, Langherans cells, fibroblasts, endothelial cells of the blood vessels) and with the membranes of the intracellular organelles (e.g. mitochondria, smooth and rough endoplasmic reticulum, nuclear membrane). As a result of this incorporation, Vitamin E modulates, as a «first messenger», several membrane signals, activating or inactivating, or however affecting intracellular metabolic pathways (second messengers) with different biochemical effects on the different types of cells which are involved (for instance, protein kinases, phosphodiesterases and cAMP activation; regulation of the entry of ionized Calcium into the cells; mast cells degranulation; regulation of the cell proliferation signals). This could, in part, explain the surprising effects which have been observed with the use of Vitamin E at mucous level.

The following are among the diseases in which therapeutic benefit has been achieved through the topical application of tocopherol acetate "as is": dryness and itching of the oral, vaginal, rectal, nasal and eye mucosa, aphthous ulcers, stomatitis, keratoconjunctivis, keratitis, keratalgia, corneal ulcers, corneal de-epithelization, encrusted rhinitis, nasal vestibulitis, atrophic vaginitis, cervical ectropion, follicular vulvitis, erythematous vulvitis, radiation-related vulvitis, genital herpes, pruritus ani, fecal incontinence, ulcerous proctitis, rectal prolapse and genital herpes.

Tocopherol and its derivatives, in particular tocopherol acetate, when used as such for topical application do not pose any problems as far as microbial contamination and conservation are concerned, in that microorganisms cannot proliferate in pure tocopherol or tocopherol derivatives. In cases in which, however, the health authorities of any country require the addition of preservatives and antibacterial agents, this can of course be carried out according to the conventional procedures of the pharmaceutical technology.

Tocopherol acetate may also be used in combination with antibiotics or antimycotics, when an infection due to micro-organisms is present, or with other active principles, such as steroidal and non-steroidal anti-inflammatory drugs, hormones, etc.

With regard to topical application, this may be performed by bringing the tocopherol acetate - possibly pre-cooled to refrigerator temperature - into direct contact with the mucosa, or through the use of vaginal or rectal suppositories, alone or in association with the above-mentioned active principles.

Suppositories may be prepared through the use, for example, of a gelatin- and glycerin-based vehicle, such as that listed in the F.U. (Italian Pharmacopoiea) VIII (water, glycerin, gelatin, 25%, 65% and 10% respectively).

### Modes for Carrying Out the Invention

Following are several examples of topical application of tocopherol acetate "as is" on various types of mucosa.

### MUCOSA OF THE ORAL CAVITY

In the oral cavity and on the lips, tocopherol acetate has been used "as is" - that is, not diluted with other components - in various pathological situations including dryness of the oral mucosa. Examples of such conditions pathological are recurrent aphthous ulcers, stomatitis and glossitis.

### Recurrent aphthous ulcers

Recurrent aphthous ulcers are the most common lesions of the oral mucosa and affect 10-30% of the population. The exact cause is unknown, though many possible etiological factors have been taken into consideration, such as iron deficiency, folic acid deficiency, vitamin B12 deficiency, viral and bacterial infections (especially of Streptococcus sanguis and mitis). There are, however, important predisposing factors, such as traumas, endocrine disorders, emotional stress, and allergies. In women, aphthous ulcers may appear cyclically, some days before the menstrual period. The theory has recently been advanced that humoral and cellulo-mediated immunity to mucosal antigens plays a major role in the determinism of the lesions.

Aphthous ulcers may be divided into 3 types, relative to their clinical characteristics: minor, major and herpetiform.

Ulcers in Behcet's syndrome should be kept in mind in the differential diagnosis, because they very often appear before the other characteristic clinical manifestations of the syndrome.

### Minor aphthous ulcers

Minor aphthous ulcers are the most common clinical form. They are small (2-6 mm in diameter), oval, very painful, covered with a yellowish-white membrane (necrotic tissue), well delimited and surrounded by an erythematous ring. They may be single or multiple (from 2 to 6), generally last for 5-8 days, then gradually heal without scarring.

They frequently recur at intervals of 1-5 months and almost always affect the "mobile" oral mucosa (buccal mucosa, lips, tongue, mucolabial and mucobuccal folds), and are very rare in the hard palate and in the gum.

Vitamin E acetate was used in 50 cases of minor aphthous ulcers in subjects aged between 8 and 40 years. It was topically applied to the involved mucosa, 3-4 times daily (quantities of 0.2-0.3 ml per application) for several days. The most important applications for therapeutic purposes were those performed immediately before going to bed for the night (and for afternoon naps, where applicable), as on such occasions the medicament remained in prolonged contact with the aphtha. All patients were advised to apply the tocopherol acetate at a temperature of about 4°C (refrigerated), as it is thicker and more easily applied at this temperature.

In all cases, subjective (reduced burning and pain) and objective benefit was observed, and the healing period was shortened (3-6 days instead of 8-10 days).

### Stomatitis.

Stomatitis is an inflammation of the mucosa of the oral cavity, with various causes.

Stomatitis medicamentosa is caused by hypersensitization to systemically administered medications (such as antipyretics, sulfonamides, antibiotics, barbiturates).

Burn-related stomatitis is linked to damage caused by heat (such as food, cutlery, boiling liquids) or by caustic chemical agents (such as during dental treatment).

Allergic stomatitis is caused by allergy to topically applied substances (such as acrylic resins and eugenol, applied during dental treatment).

Denture stomatitis is linked to repeated localized traumas.

Chemical stomatitis is caused by contact with irritants (such as applications of concentrated alcohol or of acetylsalicylic acid in case of toothache; application of eugenol and acrylic resin during dental treatment).

Radiation-related stomatitis results from radiotherapy in malignant tumors of the oral cavity, head and neck.

Chemotherapy-related stomatitis is a major complication of oncological chemotherapy.

Tocopherol acetate was used in 50 cases of stomatitis of various origins. 2-3 applications per day were made for several days, using refrigerated tocopherol acetate at a temperature of 4°C, as it is denser and more easily applied at this temperature. The application was made directly onto the lesions in the anterior part of the oral cavity (0.2-0.3 ml per application) or with a teaspoon (2 drops per application) for lesions in the posterior area of the oral cavity, especially before going to bed for the night and for afternoon naps when applicable.

The application resulted in both subjective (reduction of pain) and objective benefit (acceleration of healing).

Viral stomatitis is caused by viruses; among these, the most important and meaningful from the clinical and epidemiological point of view are those caused by herpes viruses.

The latter may be divided into primary herpetic gingivostomatitis, secondary herpetic stomatitis, herpes labialis, herpes zoster and chickenpox. Other viruses can also cause stomatitis; noteworthy among these are Coxsackie viruses and Echo viruses, which can cause herpangina or lesions of the anterior oral cavity.

Tocopherol acetate was used in 50 cases of viral stomatitis in children aged between 1 and 10 years. It was applied cold (refrigerator temperature, around 4°C) directly onto the involved areas of the interior oral cavity (twice a day, preferably before going to bed at night and also before afternoon naps in small children, in quantities of 0.2-0.3 ml per application) and administered by means of a teaspoon (2 drops twice a day) for the involved areas which were located in the posterior part of the oral cavity.

The application thus performed resulted in both subjective and objective benefit, with reduction of crying episodes, increased food intake, reduction of the number of times the children woke in the night, and accelerated healing.

### EYE MUCOSA

Tocopherol acetate was used on eye mucosa in various pathological situations characterized by a deficit of or changes in the lachrymal film (dyslachrymia) or by a de-epithelization of the conjunctival or corneal epithelium, on the assumption that tocopherol could prevent the evaporation of the lachrymal film and facilitate the re-epithelization of the conjunctiva and the cornea.

The lachrymal film, though not being part of the corneal structure, is closely associated with it, and contributes to preserving the anatomical and functional integrity of the epithelium.

It forms a smooth optical surface above the cornea, thus preventing possible changes of the image; as it is in contact with the atmosphere, it constitutes the primary source of oxygen for the cornea, which is not vascularized. In addition, it contains numerous substances with anti-bacterial properties and growth factors produced by the tear glands and involved in the physiological processes of corneal repair. Finally, it washes the ocular surfaces, carrying away exfoliated cells and foreign bodies.

In the lachrymal film, which is about 7 pm thick, three layers may be distinguished. The most internal, in direct contact with the corneal surface, is made of mucopolysaccharides; the middle layer is aqueous; the external layer is lipidic.

The external lipidic layer of the lachrymal film has two main functions: to slow the evaporation of the underlying aqueous component and to lubricate the eyelids as they move over the eyeball.

The corneal epithelium is a composite non-keratinized pavement epithelium composed of a superficial layer of squamous cells, an intermediate layer of alar cells (whose name is due to their alar, i.e. winglike extensions) and a single layer of basal columnar cells.

The corneal epithelium, in direct contact with the outside atmosphere, not only serves as a barrier to pathological agents in the environment, but must also be capable of rapid regeneration, being easily exposed to injuries of various kinds. Following an injury of any kind which causes continuous dissolution of the corneal epithelium without involvement of the Bowman's membrane, a re-epithelization process occurs by which healing is achieved in 4-7 days.

If the damage involves the entire corneal surface and extends beyond the limbus, different repair mechanisms become evident: the destruction of the stem cells at the level of the basal epithelium of the limbus promotes the growth of the conjunctival epithelium onto the cornea, with consequent corneal neovascularization.

Superficial corneal de-epithelization is a rather frequent event. Air pollution, microclimate, the use of contact lenses, and ocular dryness syndromes are the principal causes of this pathology.

In general, corneal de-epithelizations are treated with medicaments in the form of eye drops or ointments which should promote re-epithelization and relieve pain. The most common medicaments are xanthopterin, retinol, carboxymethylcellulose, hyaluronic acid, N-acetylcysteine.

None of these substances, however, has effectively demonstrated a specific re-epithelizing effect.

The search for new therapeutic principles in this connection is therefore an urgent necessity for ophthalmologists.

The use of tocopherol acetate has been regarded as useful, on the assumption that its application could prevent the evaporation of the lachrymal film, restore its lipidic component (replacing or combining with the external lipid layer of the film), lubricate the eyelids as they move over the eyeball, and promote corneal re-epithelization.

In conclusion, tocopherol acetate would behave like a kind of "artificial tear".

It should be noted that all ointments can be slightly annoying, because they temporarily reduce the flow of lachrymal fluid when the eye is opened and closed, and can cause a temporary reduction of vision.

Based on these theoretical considerations, tocopherol acetate was topically applied in various pathological conditions of the eye, and, surprisingly, gave rise to considerable subjective benefit, with marked relief of symptoms, and objective benefit, with clinical improvement till to healing.

Examples of such pathological conditions include: dyslachrymia, keratoconjunctivitis sicca, filamentous keratoconjunctivitis, iatrogenic keratoconjunctivitis, neuroparalytic keratitis, seasonal keratoconjunctivitis, recurrent keratalgia, corneal de-epithelization due to corneal foreign bodies, corneal ulcers caused by chemical agents, de-epithelization in photorefractive keratectomy and in lamellar keratoplasty.

### Keratoconjunctivitis sicca, filamentous keratoconjunctivitis, iatrogenic keratoconjunctivitis.

This includes a number of clinical conditions due to insufficient or altered production of tears. Due to the close relationship between the corneal surface and the lachrymal film, the occurrence of superficial corneal lesions due to ocular dryness may be attributed both to a deficiency in the production of one of the components of tears and to changes in the morphology of the corneal epithelium which may affect the stability of normal lachrymal film, with consequent desiccation of the ocular surface, epithelial damage and ulceration.

Vitamin E acetate was topically applied to the eye 3-5 times daily in quantities of 0.2 ml per application in 5 cases of simple keratoconjunctivitis sicca, in 5 cases of epitheliopathy due to development of scarifying changes in the eyelids, in 3 cases of epitheliopathy due to paralysis of the VII cranial nerve, and in 5 cases of nocturnal ocular dryness (in which it was applied only at night).

In all these cases the simple topical application of tocopherol acetate resulted in benefit. Within 48 hours, symptoms including pain, feeling of foreign bodies, photophobia and tearing, associated with conjunctival hyperemia and marked blepharospasm, disappeared. Examination by slit-lamp showed re-epithelization of fluorescin-positive areas after only 24-48 hours. As this pathology was chronic, the application was continued for several months to two years. The clinical benefit manifested from the beginning of treatment continued throughout that time. None of the patients treated showed any allergy to tocopherol acetate.

### Keratitis due to exposure to physical agents.

Keratitis due to exposure to physical agents may be caused by such diverse factors as trichiasis, ultraviolet radiation, foreign bodies and contact lenses.

Vitamin E acetate was topically applied to the eye in 30 cases of exposure keratitis, 3-4 times daily (0.2 ml per application) for three days. In all cases, the symptoms disappeared after the first applications and the keratitis was healed in three days of treatment.

In trichiasis, the treatment was prolonged on a perennial basis to prevent possible relapses.

### Neuroparalytic keratitis.

In 5 cases of corneal ulcer due to paralysis of the V cranial nerve, topical application of pure tocopherol acetate to the eye (3-4 times daily, 0,2 ml per application) resulted in complete re-epithelization of the ulcer within 7 days. In these cases, Vitamin E acetate was combined with a cover antibiotic (ofloxacin) until the ulcer was resolved; subsequently, Vitamin E acetate was topically applied on a continuous basis 2-3 times daily, to avoid recurrence.

### Seasonal keratoconjunctivitis.

In 5 cases of seasonal keratoconjunctivitis with corneal ulceration, the topical application of tocopherol acetate (3-4 times daily, 0.2 ml per application), associated with local corticosteroid therapy and acetylsalicylic acid per os, was of benefit, improving the symptoms linked to the ulcerative lesions, the blepharospasm and the dense mucosal secretion, and accelerating the healing of the ulcerative lesion, which healed in only 5 days.

The therapy was continued unchanged for three days more after ulcer healing. Then it was prosecuted for 6 months ( from April to September) in this way: topical application of tocopherol acetate in association with a topical antihistaminic agent three times a day for 7-10 days and twice a day for 20-30 days, then topical application of the antihistaminic agent twice a day and of tocopherol acetate only once a day in the evening before going to bed.

In four cases of seasonal keratoconjunctivitis, in the absence of ulceration but in the presence of «Roman pavement» papillae, the topical application of tocopherol acetate (0.2 ml each dose) in association with local corticosteroid therapy three times a day for 15 days, followed by topical application of tocopherol acetate in association with a topical antihistaminic agent at progressively decreasing doses, led to a significant reduction of the papillae in three cases and to total disappearance thereof in one case, in 6-7 months.

The treatment brought about disappearance of the feeling of «sand in the eye» and of the eye pain in the morning on awaking, with observation of a marked subjective and also objective (conjunctiva reddening, size of the «Roman pavement» papillae) improvement at the medical checks carried out from April to September.

### Recurrent keratalgia.

Vitamin E acetate was used in 5 cases of recurrent keratalgia following mechanical de-epithelization of the altered epithelium. it was applied 3 times daily (0.2 ml per application) for 4 days, associated with topical NSAID (non-steroid anti-inflammatory drugs). The pronounced painful symptoms were seen to disappear after the first applications, with corneal re-epithelization taking place after three days of treatment. The treatment was then continued for an additional five days, applied only at night.

### Corneal ulceration caused by chemical agents.

The topical application of Vitamin E acetate in this eye pathology gave rise to surprising results.

It was used (3-4 times daily, 0.2 ml per application, for 5-6 days and then only once, in the evening, for 3-4 days after re-epithelization had occurred) in 5 cases of corneal ulcers due to caustic substances: 1 due to contact with ammonia, 2 with sulfuric acid, 2 with caustic soda.

Immediately after the first application, the subjective symptoms of intense burning and "sand in the eyes" were reduced; within 48 hours, complete re-epithelization of the extensive area of de-epithelization had taken place, in the three cases of ammonia and sulfuric acid burns. In the caustic soda burn cases, one case, where the basal membrane was not involved, showed re-epithelization in 4 days, while the other case, although the symptoms improved, did not show any repair within three days; in that case, surgical treatment was performed.

### De-epithelization in photorefractive keratectomy and lamellar keratoplasty.

In photorefractive keratectomy, the epithelium was removed prior to the photoablative treatment: topical application of Vitamin E acetate (4 times daily, 0,2 ml per application for 4-5 days, then decreasing applications for an additional 5 days down to a single application in the evening, which was continued on a perennial basis) achieved corneal re-epithelization in three days in the 5 treated cases. In this eye pathology as well, the subjective disturbances were seen to be surprisingly alleviated.

In lamellar keratoplasty, the epithelium was totally regenerated, with repair processes which may have been modified by the primary pathology which required the intervention. Especially in lamellar keratoplasty due to vascularized leukomas following chemical burns, the application of Vitamin E acetate (4 times daily, 0,2 ml per application for 4-5 days, then decreasing applications for an additional 5 days down to a single application in the evening, which was continued on a perennial basis) produced surprising results, both in the immediate post-operative period, promoting more rapid re-epithelization, and in cases of de-epithelization which occurred in the later post-operative period, due to changes in lachrymation due to scarring.

Further to the above pathologies, the topical application of tocopherol acetate proved to be of great benefit also in various conditions of dryness and itching of the eye mucosa.

### VAGINAL MUCOSA AND VULVAR MUCO-CUTANEOUS JUNCTION

At the level of the vaginal mucosa and the muco-cutaneous vulvar zone, tocopherol acetate was used in various pathological situations for its emollient, protective and lubricant properties (as an ointment), its healing and normalizing properties with regard to the altered epithelium (as a membrane molecule and an anti-oxidant agent), and its capability of re-equilibrating the vulvo-vaginal pH (as a weak acid).

Examples of these pathologies are described below.

### Atrophic vaginitis.

Atrophic vulvovaginitis presents exclusively in old women, in whom the vagina shows signs of physiological involution caused by a progressive hormonal deficiency. This gives rise to reduced elasticity of the vaginal walls and extreme fragility of the mucosa, which becomes thinner and bleeds easily at the slightest trauma, with the appearance of punctiform hemorrhaging. Symptoms include a burning sensation and vaginal dryness, bringing about dyspareunia.

Tocopherol acetate was topically applied, by virtue of its possible protective effect and capability of improving the trophism of the vaginal mucosa, as well of restoring the ideal acidity for the vaginal ecosystem (the presence of acetic acid in the tocopherol acetate molecule makes that molecule weakly acidic).

Tocopherol acetate was applied to the vagina in quantities of 2 ml, 2 times daily (every 12 hours) in 12 old women suffering from atrophic vaginitis, for 30-40 days. In all cases treated, a recovery of the trophism of vaginal tissue was noted at the end of the treatment, accompanied by change in color (from pale to pink), increased elasticity of the tissue, and disappearance of the punctiform hemorrhaging.

In addition, all cases showed a reduction in dyspareunia (painful sexual relations); however, this reduction was significant in only half of the cases, leading even to the complete disappearance of pain during sexual relations.

### Cervical ectropion.

The term "ectropion" indicates the presence of the glandular endocervical epithelium on the external cervix. This is a physiological process which appears to result from the effects of hormonal factors, which cause hypertrophy and hyperplasia of the endocervical epithelium.

The glandular epithelium is much less resistant to traumas and infections than the squamous epithelium which covers the exocervix and the vagina; accordingly, this process can cause cervicitis. Infection of the ectropion is facilitated by the large quantities of germs present within the vagina and by the acidity of the vaginal pH.

The study was conducted on 5 young women whose average age was 22 years (18-30) and who suffered from cervical ectropion. All of the women underwent a colpocytological examination, vaginal tampon test and colposcopy, to rule out STDs and precancerous lesions. Tocopherol acetate in quantities of 2 ml, spread on a surface of 2.5 cm2 made of spongy material (Spongostan), was applied in contact with the uterine cervix three times a week for 15 days, in order to evaluate the pharmacological effects on the inflammatory aspects of the ectropion.

The colposcopy, performed before and after the treatment, showed a marked reduction in cervical congestion and in wideness of the ectropion, with early initial peripheral squamous metaplasia.

### Follicular vulvitis.

This disease results from an inflammatory process of the hair follicles and the perifollicular tissue. It exclusively involves the hair-bearing vulvar area, causing the appearance of small papules, separated by normal skin.

Tocopherol acetate was topically applied (1 ml, 2 times daily) in 5 cases, with significant clinical improvement after only a few applications.

### Erythematous vulvitis.

This disease is characterized by intense reddening and edema of the vulvar region. This form can easily involve the skin around the genito-crural and intergluteal folds. Symptoms include extreme itching and burning. This is primarily caused by trauma (cycling, horseback riding, etc.) or allergy (soaps, sanitary towels, pantyhose, etc.).

Tocopherol acetate was topically applied (1 ml, 2 times daily, for several days) in 20 cases of erythematous vulvitis. After only a few applications, a marked reduction of symptoms was achieved, with clinical normalization within 48-72 hours.

### Radiation-related vulvitis.

This may be seen following direct irradiation of the vulva, during radiotherapy. Radiodermitic lesions may vary from simple erythema to third-degree burns, with ulceration.

Tocopherol acetate, topically applied (1 ml, 2 times daily) may present valid medical assistance, by virtue of its protective, barrier, relieving and re-epithelizing effects.

### Vulvar vestibulitis.

This is also referred to as micropapillar vulvitis. It is a subclinical papovavirus infection. The velvety appearance of the medial surface of the labia minora is due to hyperplasia and hypertrophy of the mucosa. The locally cell-mediated immune deficiency promotes mycotic secondary infection. Symptoms include itching and vulvodinia.

Of the 25 women treated with tocopherol acetate (2 ml, twice daily for two weeks) , locally applied to the vestibular vulvar mucosa, about 90% experienced complete remission of itching and vulvar pain, while the remaining 10% showed no improvement of symptoms.

The barrier and antioxidant effects of tocopherol acetate, which relieves the inflammatory process (edema, reddening) and re-balances the pH of the vestibular mucosa, inhibits the development of the mycotic process, and thus promotes the disappearance of itching and vulvodinia.

### RECTAL MUCOSA AND ANAL MUCO-CUTANEOUS JUNCTION

Tocopherol acetate was topically applied in various pathological conditions of the anal mucosa, perianal region and in proctitis.

### Pruritus ani

Seven patients suffering from pruritus ani, in whom no visible organic cause was manifest upon proctological examination, were treated with tocopherol acetate (0,2-0,4 ml applied twice daily for 30 days.

At the end of the treatment, 4 patients experienced a complete resolution of symptoms, and the remaining 3 patients showed a satisfactory reduction.

### Fecal incontinence

There are two types of fecal incontinence: minor (or partial or occasional) and major. In the latter case, not even well-formed feces can be retained.

10 patients suffering from minor fecal incontinence and 5 suffering from major fecal incontinence were treated with tocopherol acetate (1-2 ml three times a day for 30 days).

At an objective exam, there was a constant and considerable reduction of inflammation, apparently linked to the protection of the skin against the irritating feces.

The benefit noted by the patients suffering from the minor form was especially noteworthy, whereas the results signalled by patients suffering from the major form were good.

### Ulcerous proctitis

Ulcerous proctitis is a variety of ulcerous colitis exclusively located in the rectum; it can also be caused by sexual practices or treatment with ionizing radiation.

Characteristics of ulcerous proctitis include mild proctorrhagia with issue of bright red blood, stypsis, and cramping pains in the left quadrants.

Five patients suffering from proctitis following radiotherapy were treated with tocopherol acetate; the tocopherol acetate was given in quantities of 0.8 ml in microsuppositories twice a day for 30 days.

All patients experienced benefit, from slight to good. Endoscopic examination revealed improvement of the general aspect, with absence of erosive lesions and reduced fragility, although a hypotrophic condition was still present.

### Rectal prolapse

Five patients suffering from rectal prolapse, associated with inflammation, erosions and rhagades of the mucosa, were treated with Vitamin E acetate (0,6-0,8 ml three times per day) for one month.

The subjective results were excellent as early as the first few days, with significant reduction of pain, burning and annoyance.

Objectively, examination after 15 days showed significant reduction of inflammation and disappearance of the mucosal erosions and rhagades.

The treatment, which was continued in all subjects for many months (up to two years), enabled the preservation of the results obtained, with no reappearance of inflammation or erosions of the prolapsed mucosa.

Finally, the topical application of tocopherol acetate provided significant relief in several conditions of dryness and itching of the rectal mucosa.

### NASAL MUCOSA AND NASAL VESTIBULAR MUCO-CUTANEOUS JUNCTION

At the level of the nasal mucosa as well, it was shown that the topical application of tocopherol acetate can be of use in various pathological situations, some of which are described by way of example.

### Encrusted rhinitis.

In many inflammatory processes of the nasal cavities, of various origins and kinds, crusted secretions or sero-hematic clots are present in the cavities themselves. These diseases include infectious rhinitis, mild recurrent epistaxis (nosebleed), status post adenoidectomy, microtraumas (fingers in the nose, especially in children).

In 50 cases of encrusted rhinitis, the application of tocopherol acetate (0.2 ml, 2-3 times daily) for 2-4 days was shown to be beneficial, promoting the disappearance of the crusts and/or clots and the protection of the nasal mucosa.

### Eczematous nasal vestibulitis, with rhagades.

Eczema of the nasal vestibulum, with frequent formation of rhagades, is a consequence of rhinitic processes, acute or chronic, whose exudate irritates the cutaneous lining of the vestibulum.

In 100 cases of vestibular eczema (almost all as a result of the common cold), the application of Vitamin E acetate, with its emollient, protective and re-epithelizing effects, caused the disappearance of the rhagades in 48 hours and the normalization of the eczematous skin in 3-6 days.

### Epistaxis

Epistaxis is due to rupture of the blood vessels of the nasal mucosa owing to different causes.

Epistaxis can be treated with a causal therapy, when possible, and with a local therapy suited to the importance thereof.

In cases of recurrent slight epistaxis, the topical application of tocopherol acetate appears to be useful as an adjuvant treatment, thanks to its emollient, protective and eutrophic action.

Tocopherol acetate was used (0.2 ml 2-3 times a day for 15 days) in 20 children suffering from slight epistaxis due to traumas connected with the bad habit of repeatedly introducing fingers in the nose cavities. In all cases disappearance of the epistaxis episodes was achieved.

### Locus Valsalvae varices

The varices of the Locus Valsalvae are a frequent cause of epistaxis, especially in the children. Caustication of these varices is not often performed on little children due to the risk of a iatrogenic perforation of the nasal septum.

Tocopherol acetate was applied topically (0.2 ml 2-3 times a day) for 3 months on the mucosa of the nasal septum in 60 children (aged between 2 and 6 years) suffering from Locus Valsalvae varices and recurrent epistaxis. In all cases a reduction, up to disappearance, of the epistaxis episodes was achieved during the treatment period and the medical checks revealed an improvement of the trophism of the nose mucose and of the macroscopic aspect of the varices themselves.

Tocopherol acetate, when topically applied, appears to exert an eutrophic and protective activity on the epithelium of the nasal mucosa, which is important for preventing those irritations that, particularly in children, can lead to scratching and nose bleeding even more so in the presence of local varicose alterations. Such an eutrophic and protective activity proved to be of benefit also in all those conditions characterized by dryness and itching of the nasal mucosa.

### Ulcerations of the nose mucosa from local foreign bodies

Children often introduce into their nose foreign bodies of different origin which cause ulcerative lesions of the nasal mucosa.

Tocopherol acetate (0.2 ml, 2-3 times a day) was applied topically for 7 days to 15 children suffering from nasal ulcers due to foreign body. In all cases disappearance of the ulcerations was noticed at the control after 7 days of treatment.

The effect of topical application of tocopherol acetate was also noteworthy in cases of genital herpes.

### Genital herpes

Vitamin E acetate was used for topical application by 20 patients (10 men and 10 women) suffering from genital herpes (aged between 16 and 45 years).

In 17 cases, a marked improvement was obtained with the application of Vitamin E acetate at least three times daily, in quantities of 0.2-0.4 ml each time.

When Vitamin E acetate was applied to the first signs of the disease (itching, paresthesia or burning, initial vesicles), it was noted that the vesicles failed to appear (if initially absent), or that they developed into the encrusted phase within 1-3 days (if initially present), with decided acceleration of recovery.

When Vitamin E acetate was applied in herpetic diseases which were already well-established (multiple vesicles present for 1-2 days), no new vesicles appeared and the existing lesions were seen to stop growing, with accelerated development into the encrusted phase (1 or 2 days).

Moreover, immediately after the application, the subjective symptoms of burning and/or itching associated with the herpetic manifestation disappeared.

Only in one case did the application of Vitamin E acetate not modify the normal course of the herpetic manifestation and not lead to attenuation of the subjective symptoms associated therewith.

In 2 cases, the lack of clinical response to the application of Vitamin E acetate seemed to be linked to insufficient frequency of application,-because when the number of applications was increased in the course of the day, recovery was speeded.

## Claims

1. The use of a compound selected from the group consisting of Vitamin E and esters thereof for the preparation of a medicament for the topical treatment of at least one condition, chosen from genital herpes, epistaxis and pathologies affecting the eye mucosa, the rectal mucosa and the anal muco-cutaneous junction.

2. The use as claimed in Claim 1, wherein said compound is an ester of Vitamin E with a carboxylic acid of formula R-COOH, wherein R is an alkyl, alkenyl or alkinyl residue having 1 to 19 carbon atoms.

3. The use as claimed in Claim 2, wherein said carboxylic acid is selected from the group consisting of acetic acid, n-propionic acid, succinic acid and linoleic acid.

4. The use as claimed in Claim 3, wherein said compound is Vitamin E acetate.

5. The use according to any one of Claims 1-4, wherein said compound is the sole active substance used in the preparation of said medicament.

6. The use as claimed in Claim 5, wherein said compound is the only ingredient used in the preparation of said medicament.

7. The use according to any one of Claims 1-4, wherein at least one further active substance is used in addition to said compound in the preparation of said medicament.

8. The use according to any one of claims 1-7, wherein said pathologies include dyslachrymia, keratoconjunctivitis, keratitis, keratalgia, corneal ulcers, corneal de-epithelization, dryness and itching of the eye mucosa.

9. The use according to any one of claims 1-7, wherein said pathologies include pruritus ani, fecal incontinence, proctitis, ulcerous proctitis, rectal prolapse, dryness and itching of the rectal mucosa.

10. The use of a compound selected from the group consisting of Vitamin E and esters thereof as the sole active substance for the preparation of a medicament for the topical treatment of pathologies affecting the oral mucosa and the vaginal mucosa.

11. The use according to Claim 10, wherein said compound is an ester of Vitamin E with a carboxylic acid of formula R-COOH, wherein R is an alkyl, alkenyl or alkinyl residue having 1 to 19 carbon atoms.

12. The use according to Claim 11, wherein said carboxylic acid is selected from the group consisting of acetic acid, n-propionic acid, succinic acid and linoleic acid.

13. The use according to Claim 12, wherein said compound is Vitamin E acetate.

14. The use according to any one of Claims 10 to 13, wherein said compound is the only ingredient used in the preparation of said medicament.

15. The use according to any one of claims 10 to 14, wherein said pathologies include aphthous ulcers, stomatitis, glossitis and dryness and itching of the oral mucosa.

16. The use according to any one of claims 10 to 14, wherein said pathologies include atrophic vaginitis, cervical ectropion, dryness and itching of the vaginal mucosa.

17. A pharmaceutical composition for the topical treatment of at least one condition chosen from genital herpes and pathologies affecting the eye mucosa, the rectal mucosa and the anal muco-cutaneous junction, consisting of a compound selected from the group of Vitamin E and the esters thereof and at least one active substance selected from steroidal and non-steroidal anti-inflammatory agents, antibiotics, antimycotics and hormones.

18. A composition as claimed in Claim 17, wherein said compound is Vitamin E acetate.

19. A pharmaceutical composition for the treatment of pathologies affecting the eye mucosa comprising a pharmaceutically acceptable carrier, one compound selected from the group consisting of Vitamin E and the esters thereof, and optionally at least one active principle selected from the group consisting of steroidal and non-steroidal anti-inflammatory agents, antibiotics, antimycotics and hormones.

20. A pharmaceutical composition as claimed in Claim 19, wherein said compound is Vitamin E acetate.

## Patentansprüche

1. Verwendung einer Verbindung, die aus der Gruppe bestehend aus Vitamin E und dessen Estern ausgewählt ist, für die Herstellung eines Medikaments zur topischen Behandlung von zumindest einer Erkrankung gewählt aus genitalem Herpes, Epistaxis und Krankheitsbildern, die die Augenschleimhaut, die rektale Schleimhaut und den Übergang der analen Schleimhaut zur Haut betreffen.

2. Verwendung nach Anspruch 1, wobei die Verbindung ein Ester von Vitamin E mit einer Carbonsäure der Formel R-COOH ist, wobei R ein Alkyl-, Alkenyl- oder Alkinylrest mit 1 bis 19 Kohlenstoffatomen ist.

3. Verwendung nach Anspruch 2, wobei die Carbonsäure aus der Gruppe bestehend aus Essigsäure, n-Propionsäure, Bemsteinsäure und Linolsäure gewählt ist.

4. Verwendung nach Anspruch 3, wobei die Verbindung Vitamin E-Acetat ist.

5. Verwendung nach einem der Ansprüche 1 bis 4, wobei die genannte Verbindung als einzige aktive Verbindung bei der Herstellung des Medikaments verwendet wird.

6. Verwendung nach Anspruch 5, wobei die Verbindung der einzige, verwendete Bestandteil bei der Herstellung des Medikaments ist.

7. Verwendung nach einem der Ansprüche 1 bis 4, wobei zumindest eine weitere aktive Substanz zusätzlich zu der Verbindung bei der Herstellung des Medikaments verwendet wird.

8. Verwendung nach einem der Ansprüche 1 bis 7, wobei die Krankheitsbilder Dyslachrymie, Keratoconjunctivitis, Keratitis, Keratalgie, Ulcus der Hornhaut, Abbau des Hornhautepithelgewebes sowie Trockenheit und Jucken der Augenschleimhaut einschließen.

9. Verwendung nach einem der Ansprüche 1 bis 7, wobei die Krankheitsbilder Afterjucken, Stuhlinkontinenz, Proktitis, ulceröse Proktitis, Rektumprolaps sowie Trockenheit und Jucken der rektalen Schleimhaut einschließen.

10. Verwendung einer Verbindung, die aus der Gruppe bestehend aus Vitamin E und dessen Estern ausgewählt ist, als die einzige aktive Substanz für die Herstellung eines Medikaments für die topische Behandlung von Krankheitsbildern, die die Mundschleimhaut und die Vaginalschleimhaut betreffen.

11. Verwendung nach Anspruch 10, wobei die Verbindung ein Ester von Vitamin E mit einer Carbonsäure der Formel R-COOH ist, wobei R ein Alkyl-, Alkenyl- oder Alkinylrest mit 1 bis 19 Kohlenstoffatomen ist.

12. Verwendung nach Anspruch 11, wobei die Carbonsäure aus der Gruppe bestehend aus Essigsäure, n-Propionsäure, Bemsteinsäure und Linolsäure gewählt ist.

13. Verwendung nach Anspruch 12, wobei die Verbindung Vitamin E-Acetat ist.

14. Verwendung nach einem der Ansprüche 10 bis 13, wobei die Verbindung der einzige Bestandteil bei der Herstellung des Medikaments ist.

15. Verwendung nach einem der Ansprüche 10 bis 14, wobei die Krankheitsbilder Aphten, Stomatitis, Glossitis, sowie trockene und juckende Mundschleimhaut einschließen.

16. Verwendung nach einem der Ansprüche 10 bis 14, wobei die Krankheitsbilder atrophische Vaginitis, Zervixektropie sowie Trockenheit und Jucken der Vaginalschleimhaut sind.

17. Eine pharmazeutische Zusammensetzung für die topische Behandlung von zumindest einer Krankheit, die aus genitalem Herpes und Krankheitsbildern, die die Augenschleimhaut, die rektale Schleimhaut und den Übergang von der analen Schleimhaut zur Haut betreffen, gewählt ist, bestehend aus einer Verbindung, die aus der Gruppe von Vitamin E und dessen Estern ausgewählt ist, und zumindest einer aktiven Substanz ausgewählt aus steroidalen und nicht-steroidalen Entzündungshemmern, Antibiotika, Antimykotika und Hormonen.

18. Zusammensetzung nach Anspruch 17, wobei die Verbindung Vitamin E-Acetat ist.

19. Eine pharmazeutische Zusammensetzung für die Behandlung von Krankheitsbildern, die die Augenschleimhaut betreffen, die einen pharmazeutisch verträglichen Träger, eine Verbindung ausgewählt aus der Gruppe bestehend aus Vitamin E und dessen Estern, und, gegebenenfalls, zumindest einen aktiven Grundbestandteil ausgewählt aus der Gruppe bestehend aus steroidalen und nicht-steroidalen Entzündungshemmern, Antibiotika, Antimykotika und Hormone, enthält.

20. Pharmazeutische Zusammensetzung nach Anspruch 19, wobei die Verbindung Vitamin E-Acetat ist.

## Revendications

1. L'utilisation d'un composé choisi dans le groupe constitué par la vitamine E et ses esters pour la préparation d'un médicament pour le traitement topique d'au moins un état choisi parmi l'herpès génital, l'épistaxis et les pathologies affectant la muqueuse oculaire, la muqueuse rectale et la jonction muco-cutanée anale.

2. L'utilisation revendiquée dans la revendication 1, où ledit composé est un ester de vitamine E avec un acide carboxylique de formule R-COOH, où R est un résidu alkyle, alcényle ou alcynyle ayant de 1 à 19 atomes de carbone.

3. L'utilisation revendiquée dans la revendication 2, où ledit acide carboxylique est choisi dans le groupe constitué par l'acide acétique, l'acide n-propionique, l'acide succinique et l'acide linoléique.

4. L'utilisation revendiquée dans la revendication 3, où ledit composé est l'acétate de vitamine E.

5. L'utilisation selon l'une des revendications 1 à 4, où ledit composé est le seul principe actif utilisé pour la préparation dudit médicament.

6. L'utilisation selon la revendication 5, où ledit composé est le seul ingrédient utilisé pour la préparation dudit médicament.

7. L'utilisation selon l'une des revendications 1 à 4, où l'on utilise au moins . un autre principe actif en plus dudit composé pour la préparation dudit médicament.

8. L'utilisation selon l'une des revendications 1 à 7, où lesdites pathologies comprennent la dyslacrymie, la kératoconjonctivite, la kératite, la kératalgie, les ulcères de la cornée, la déépithélisation de la cornée, la sécheresse et l'irritation de la muqueuse oculaire.

9. L'utilisation selon l'une des revendications 1 à 7, où lesdites pathologies comprennent le prurit anal, l'incontinence fécale, la proctite, la proctite ulcéreuse, le prolapsus rectale, la sécheresse et l'irritation de la muqueuse rectale.

10. L'utilisation d'un composé choisi dans le groupe constitué par la vitamine E et ses esters comme seul principe actif pour la préparation d'un médicament pour le traitement topique de pathologies affectant la muqueuse orale et la muqueuse vaginale.

11. L'utilisation selon la revendication 10, où ledit composé est un ester de vitamine E avec un acide carboxylique de formule R-COOH, où R est un résidu alkyle, alcényle ou alcynyle ayant de 1 à 19 atomes de carbone.

12. L'utilisation selon la revendication 11, où ledit acide carboxylique est choisi dans le groupe constitué par l'acide acétique, l'acide n-propionique, l'acide succinique et l'acide linoléique.

13. L'utilisation selon la revendication 12, où ledit composé est l'acétate de vitamine E.

14. L'utilisation selon l'une des revendications 10 à 13, où ledit composé est le seul ingrédient utilisé pour la préparation dudit médicament.

15. L'utilisation selon l'une des revendications 10 à 14, où lesdites pathologies comprennent les ulcères aphteux; la stomatite, la glossite et la sécheresse et l'irritation de la muqueuse orale.

16. L'utilisation selon l'une des revendications 10 à 14, où lesdites pathologies comprennent la vaginite atrophique, l'ectropion cervical, la sécheresse et l'irritation de la muqueuse vaginale.

17. Une composition pharmaceutique pour le traitement topique d'au moins un état choisi parmi l'herpès génital et les pathologies affectant la muqueuse oculaire, la muqueuse rectale et la jonction muco-cutanée anale, consistant en un composé choisi dans le groupe constitué par la vitamine E et ses esters et au moins un principe actif choisi parmi les agents anti-inflammatoires stéroïdiens et non stéroïdiens, les antibiotiques, les antimycotiques et les hormones.

18. Une composition telle que revendiquée dans la revendication 17, où ledit composé est l'acétate de vitamine E.

19. Une composition pharmaceutique pour le traitement de pathologies affectant la muqueuse oculaire comprenant un véhicule pharmaceutiquement acceptable, un composé choisi dans le groupe constitué par la vitamine E et ses esters et éventuellement au moins un principe actif choisi dans le groupe constitué par les agents anti-inflammatoires stéroïdiens et non stéroïdiens, les antibiotiques, les antimycotiques et les hormones.

20. Une composition telle que revendiquée dans la revendication 19, où ledit composé est l'acétate de vitamine E.
